# EUROPEAN PATENT APPLICATION

(11) **EP 4 458 825 A1**
(43) Date of publication of application: **06.11.2024**
(21) Application number: 22914529.7
(22) Date of filing: 23.12.2022
(51) Int. Cl.: C07D 413/14, A61K 31/517, A61P 11/00, A61P 31/12, A61P 9/10, A61P 35/02, A61P 37/00

(54) **SALT OF AMINOPYRIMIDINE COMPOUND AND USE THEREOF**

(30) Priority: 28.12.2021 CN 202111620875
(71) Applicant: Sunshine Lake Pharma Co., Ltd., Dongguan, Guangdong 523000 (CN)
(72) Inventor: XI, Ning, Dongguan, Guangdong 523871 (CN); LI, Xiaobo, Dongguan, Guangdong 523871 (CN); LI, Minxiong, Dongguan, Guangdong 523871 (CN); FENG, Xuejin, Dongguan, Guangdong 523871 (CN)
(74) Representative: Hoffmann Eitle
(86) International application number: PCT/CN2022/141345
(87) International publication number: WO 2023/125275

(57) **Abstract**

Provided are a salt of an aminopyrimidine compound and a use thereof. Specifically, provided are a salt of (*S*)-2-(1-((6-amino-5-(3-methyl-1,2,4-oxadiazol-5-yl)pyrimidin-4-yl)amino)ethyl)-5-(3-hydroxypro p-1-yn-1-yl)-3-phenylquinazolin-4(3*H*)-one and a pharmaceutical composition comprising the salt. Further provided is a use thereof in the preparation of a medicament for treating and preventing diseases related to PI3K kinase abnormalities.

## Description

### FIELD OF THE INVENTION

The present invention belongs to the field of medical technology, relates to a salt of an aminopyrimidine compound and a use thereof, specifically relates to a salt of (*S*)-2-(1-((6-amino-5-(3-methyl-1,2,4-oxadiazol-5-yl)pyrimidin-4-yl)amino)ethyl)-5-(3-hydroxypro p-1-yn-1-yl)-3-phenylquinazolin-4(3*H*)-one and a pharmaceutical composition containing the salt, and further relates to a use thereof in the preparation of a medicament.

### BACKGROUND ART

Phosphoinositide 3-kinases (PI3K kinases or PI3Ks), as a family of lipid kinases, play an important regulatory role in many cellular processes, such as cell survival, reproduction, and differentiation. As major effectors downstream of receptor tyrosine kinases (RTKs) and G protein-coupled receptors (GPCRs), PI3Ks transduce signals from various growth factors and cytokines into intracellular massages by generating phospholipids, which activate the serine-threonine protein kinase AKT (also known as protein kinase B (PKB)) and other downstream effector pathways. The tumor suppressor or PTEN (phosphatase and tensin homologue) is the most important negative regulator of the PI3K signaling pathway ("Small-molecule inhibitors of the PI3K signaling network". Future Med Chem., 2011, 3 (5), 549-565).

To date, eight mammalian PI3Ks have been identified, divided into three main classes (I, II and III) on the basis of their genetic sequence, structure, adapter molecules, expression, mode of activation, and preferred substrate. Among them, class I PI3Ks are further divided based on signaling pathways and regulatory proteins into class IA and class IB. The class IA PI3Ks (PI3Kα, PI3Kβ, and PI3Kδ) is a heterodimer complex composed of p110 catalytic subunits (p110α, p110β, and p110δ respectively) and p85 regulatory adapter subunits (i.e., p85α, p85β, p55δ, p55α and p50α). The catalytic p110 subunit uses ATP to phosphorylate phosphatidylinositol (PI, PtdIns), PI4P and PI (4,5) P2. These respond to signaling generally through receptor tyrosine kinases (RTKs). The class IB PI3Kγ signals through G-protein-coupled receptors (GPCRs) and is composed of a p110γ catalytic subunit, and the regulatory subunit associated with p110γ is distinct from the class IA isoform.

PI3Kδ signaling has been implicated in B cell survival, migration, and activation (Puri, Frontiers in Immunology, 2012, 3(256), 1-16, at pp. 1-5; and Clayton, J Exp Med, 2002, 196(6): 753-63). For example, PI3Kδ is required for B cell receptor-driven antigen-dependent B cell activation. By blocking B cell adhesion, survival, activation, and proliferation, PI3Kδ inhibition impairs B cells' ability to activate T cells, thereby preventing their activation and reducing the secretion of autoantibodies and pro-inflammatory cytokines. Thus, through its ability to inhibit B cell activation, PI3Kδ inhibitors will be expected to treat B cell-mediated disease that can be treated with similar approaches (e.g., B cell depletion by rituximab). In fact, PI3Kδ inhibitors have been shown to be suitable in mouse models of a variety of autoimmune diseases (such as arthritis) that can also be treated with rituximab (Puri (2012)). In addition, innate B-cells associated with autoimmunity are sensitive to PI3Kδ activity, as MZ and B-1 cells are almost absent in mice lacking the p110δ gene (Puri (2012)). PI3Kδ inhibitors reduce the trafficking and activation of MZ and B-1 cells, which are implicated in autoimmune diseases.

In addition to their potential role in inflammatory disease, all four class I PI3K subtypes have a role to play in cancer. The gene encoding p110α is frequently mutated in common cancers (including breast, prostate, colon, and endometrial cancers) (Samuels et al., Science, 2004, 304(5670): 554; Samuelset al., Curr Opin Oncol. 2006, 18(1): 77-82). Eighty percent of these mutations are represented by one of the three amino acid substitutions in the helix or kinase domain of the enzyme and result in significant upregulation of kinase activity, resulting in oncogenic transformations in cell cultures and in animal models (Kang et al., Proc Natl Acad Sci USA. 2005, 102(3): 802-7; Baderet al., Proc NatlAcad Sci USA. 2006, 103(5): 1475-9). Such mutations have not been identified in other PI3K subtypes, but there is evidence that they can promote the development and progression of malignancy. Consistent overexpression of PI3Kδ was observed in acute myeloblastic leukemia (Sujobert et al., Blood, 2005, 106(3): 1063-6) and inhibitors of PI3Kδ prevented the growth of leukemia cells (Billottet et al., Oncogene. 2006, 25(50): 6648-59). Elevated expression of PI3Kγ was observed in chronic myeloid leukemia (Hickey et al., J Biol.Chem. 2006, 281(5): 2441-50). Alterations in the expression of PI3Kβ, PI3Kγ, and PI3Kδ have also been observed in brain, colon, and bladder cancers (Benistant et al., Oncogene, 2000, 19(44): 5083-90; Mizoguchi et al., Brain Pathol. 2004, 14(4): 372-7; Knobbe et al., Neuropathol Appl Neurobiol. 2005, 31(5): 486-90).

International application WO2019143874 A1 discloses compound (*S*)-2-(1-(6-amino-5-(3-methyl-1,2,4-oxadiazol-5-yl)pyrimidin-4-yl)amino)ethyl)-5-(3-hydroxyprop yl-1-yn-1-yl)-3-phenylquinazolin-4(3*H*)-one (as indicated in formula (I)), which can treat or alleviate diseases associated with abnormal expression of PI3K kinase, such as hematologic malignancies. The compound has problems such as poor solubility, unsatisfactory absorption in animals and poor stability, which bring many inconveniences to the subsequent formulation development.

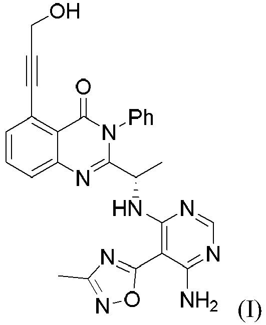

Different salt or solid forms of the active pharmaceutical ingredient may have different properties. Changes in properties of different salts or solids can provide improved formulations, for example, ease of synthesis or handling, improved dissolution, or improved stability and shelf life. Changes in properties due to different salts or solid forms can also improve the pharmacological properties of the final formulation product, for example, by increasing exposure, bioavailability, or extending half-life.

### SUMMARY

The present invention provides a salt of a compound of formula (I) Specifically, the present invention provides sulfate salt, bisulfate salt, phosphate salt and p-toluenesulfonate salt of the compounds shown in formula (I), and the salt described in the present invention has good stability and has better pharmacokinetic properties, so as to have better druggability.

Specifically, the present invention relates to salts of the compound of formula (I) and pharmaceutical compositions thereof, and the use in the preparation of drugs for the treatment or prevention of diseases associated with abnormal expression of PI3K kinase. The salt described in the present invention may also be in the form of a solvated substance, such as a hydrate form.

In one aspect, provided herein is a salt of a compound of formula (I);

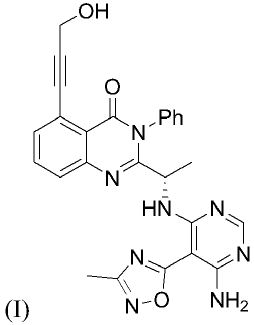

In some embodiments, the salts of the compounds shown in formula (I) of the present invention include, but are not limited to, hydrochloride salt, hydrobromide salt, phosphate salt, sulfate salt, bisulfate salt, p-toluenesulfonate salt, benzenesulfonate salt, benzoate salt, acetate salt, oxalate salt, maleate salt, tartrate salt or citrate salt, etc.

In some embodiments, the salt of the compound shown in formula (I) of the present invention is the sulfate salt of the compound shown in formula (I).

In some embodiments, the sulfate salt of the compound shown in formula (I) of the present invention is the crystal form A of the sulfate salt of the compound shown in formula (I).

In some embodiments, the X-ray powder diffraction pattern of crystal form A of sulfate salt of the compound shown in formula (I) of the present invention comprises the following diffraction peaks at 2θ angles: 5.37° ± 0.2°, 6.90° ± 0.2°, 11.82° ± 0.2°, 16.51° ± 0.2°, 20.50° ± 0.2° and 25.95° ± 0.2°.

In other embodiments, the X-ray powder diffraction pattern of crystal form A of sulfate salt of the compound shown in formula (I) of the present invention comprises the following diffraction peaks at 2θ angles: 5.37° ± 0.2°, 6.20° ± 0.2°, 6.90° ± 0.2°, 11.82° ± 0.2°, 16.51° ± 0.2°, 16.60° ± 0.2°, 19.18° ± 0.2°, 20.10° ± 0.2°, 20.50° ± 0.2°, 21.64° ± 0.2°, 25.95° ± 0.2° and 29.63° ± 0.2°.

In other embodiments, the X-ray powder diffraction pattern of crystal form A of sulfate salt of the compound shown in formula (I) of the present invention comprises the following diffraction peaks at 2θ angles: 5.37° ± 0.2°, 6.20° ± 0.2°, 6.90° ± 0.2°, 8.74° ± 0.2°, 10.77° ± 0.2°, 10.99° ± 0.2°, 11.82° ± 0.2°, 12.45° ± 0.2°, 15.61° ± 0.2°, 16.51° ± 0.2°, 16.60° ± 0.2°, 17.08° ± 0.2°, 17.57° ± 0.2°, 17.91° ± 0.2°, 18.62° ± 0.2°, 19.18° ± 0.2°, 20.10° ± 0.2°, 20.50° ± 0.2°, 20.91° ± 0.2°, 21.64° ± 0.2°, 22.24° ± 0.2°, 23.31° ± 0.2°, 23.70° ± 0.2°, 23.83° ± 0.2°, 24.41° ± 0.2°, 24.87° ± 0.2°, 25.18° ± 0.2°, 25.59° ± 0.2°, 25.95° ± 0.2°, 27.15° ± 0.2°, 27.55° ± 0.2°, 27.98° ± 0.2°, 28.19° ± 0.2°, 28.51° ± 0.2°, 29.63° ± 0.2°, 30.19° ± 0.2° , 31.83° ± 0.2°, 36.11° ± 0.2° and 41.55° ± 0.2°.

In some embodiments, the bisulfate salt of the compound shown in formula (I) of the present invention is the crystal form A of the bisulfate salt of the compound shown in formula (I).

In some embodiments, the X-ray powder diffraction pattern of crystal form A of bisulfate salt of the compound shown in formula (I) of the present invention comprises the following diffraction peaks at 2θ angles: 6.49° ± 0.2°, 6.81° ± 0.2°, 11.54° ± 0.2°, 13.79° ± 0.2°, 21.99° ± 0.2°, 25.99° ± 0.2° and 26.32° ± 0.2°.

In other embodiments, the X-ray powder diffraction pattern of crystal form A of bisulfate salt of the compound shown in formula (I) of the present invention comprises the following diffraction peaks at 2θ angles: 6.49° ± 0.2°, 6.81° ± 0.2°, 8.75° ± 0.2°, 11.54° ± 0.2°, 13.06° ± 0.2°, 13.64° ± 0.2°, 13.79° ± 0.2°, 16.24° ± 0.2°, 17.11° ± 0.2°, 21.99° ± 0.2°, 25.99° ± 0.2°, 26.32° ± 0.2°, 26.90° ± 0.2° and 27.91° ± 0.2°.

In other embodiments, the X-ray powder diffraction pattern of crystal form A of bisulfate salt of the compound shown in formula (I) of the present invention comprises the following diffraction peaks at 2θ angles: 6.49° ± 0.2°, 6.81° ± 0.2°, 8.75° ± 0.2°, 11.54° ± 0.2°, 13.06° ± 0.2°, 13.64° ± 0.2°, 13.79° ± 0.2°, 14.19° ± 0.2°, 14.70° ± 0.2°, 16.24° ± 0.2°, 17.11° ± 0.2°, 18.10° ± 0.2°, 18.59° ± 0.2°, 19.48° ± 0.2°, 19.68° ± 0.2°, 21.20° ± 0.2°, 21.52° ± 0.2°, 21.99° ± 0.2°, 23.25° ± 0.2°, 24.05° ± 0.2°, 24.57° ± 0.2°, 25.57° ± 0.2°, 25.99° ± 0.2°, 26.32° ± 0.2°, 26.90° ± 0.2°, 27.08° ± 0.2°, 27.91° ± 0.2°, 28.61° ± 0.2°, 29.48° ± 0.2°, 31.02° ± 0.2°, 32.88° ± 0.2°, 33.53° ± 0.2°, 35.20° ± 0.2°, 38.45° ± 0.2°, 39.52° ± 0.2°, 40.58° ± 0.2°, 41.72° ± 0.2° and 43.81° ± 0.2°.

In some embodiments, the salt of the compound shown in formula (I) of the present invention is the phosphate salt of the compound shown in formula (I).

In some embodiments, the phosphate salt of the compound shown in formula (I) of the present invention is the crystal form A of the phosphate salt of the compound shown in formula (I).

In some embodiments, the X-ray powder diffraction pattern of crystal form A of phosphate salt of the compound shown in formula (I) of the present invention comprises the following diffraction peaks at 2θ angles: 7.41° ± 0.2°, 11.06° ± 0.2°, 11.27° ± 0.2°, 16.62° ± 0.2°, 19.32° ± 0.2°, 22.27° ± 0.2° and 24.38° ± 0.2°.

In other embodiments, the X-ray powder diffraction pattern of crystal form A of phosphate salt of the compound shown in formula (I) of the present invention comprises the following diffraction peaks at 2θ angles: 7.41° ± 0.2°, 9.27° ± 0.2°, 11.06° ± 0.2°, 11.27° ± 0.2°, 12.02° ± 0.2°, 13.30° ± 0.2°, 16.62° ± 0.2°, 19.32° ± 0.2°, 20.69° ± 0.2°, 22.27° ± 0.2°, 24.38° ± 0.2°, 26.58° ± 0.2°, 27.87° ± 0.2° and 28.32° ± 0.2°.

In other embodiments, the X-ray powder diffraction pattern of crystal form A of phosphate salt of the compound shown in formula (I) of the present invention comprises the following diffraction peaks at 2θ angles: 5.02° ± 0.2°, 5.95° ± 0.2°, 7.41° ± 0.2°, 8.15° ± 0.2°, 9.27° ± 0.2°, 9.93° ± 0.2°, 11.06° ± 0.2°, 11.27° ± 0.2°, 12.02° ± 0.2°, 12.82° ± 0.2°, 13.30° ± 0.2°, 14.87° ± 0.2°, 16.62° ± 0.2°, 17.09° ± 0.2°, 18.11° ± 0.2°, 19.32° ± 0.2°, 19.79° ± 0.2°, 20.69° ± 0.2°, 21.22° ± 0.2°, 22.27° ± 0.2°, 23.41° ± 0.2°, 24.38° ± 0.2°, 25.47° ± 0.2°, 26.58° ± 0.2°, 27.43° ± 0.2°, 27.87° ± 0.2°, 28.32° ± 0.2°, 29.45° ± 0.2°, 30.34° ± 0.2°, 31.62° ± 0.2° and 33.79° ± 0.2°.

In some embodiments, the salt of the compound shown in formula (I) of the present invention is the p-toluenesulfonate salt of the compound shown in formula (I).

In some embodiments, the p-toluenesulfonate salt of the compound shown in formula (I) of the present invention is the crystal form A of the p-toluenesulfonate salt of the compound shown in formula (I).

In some embodiments, the X-ray powder diffraction pattern of crystal form A of p-toluenesulfonate salt of the compound shown in formula (I) of the present invention comprises the following diffraction peaks at 2θ angles: 6.49° ± 0.2°, 10.04° ± 0.2°, 11.71° ± 0.2°, 14.10° ± 0.2°, 17.86° ± 0.2°, 20.84° ± 0.2° and 21.87° ± 0.2°.

In other embodiments, the X-ray powder diffraction pattern of crystal form A of p-toluenesulfonate salt of the compound shown in formula (I) of the present invention comprises the following diffraction peaks at 2θ angles: 6.49° ± 0.2°, 9.51° ± 0.2°, 10.04° ± 0.2°, 10.51° ± 0.2°, 10.72° ± 0.2°, 11.71° ± 0.2°, 14.10° ± 0.2°, 14.94° ± 0.2°, 16.82° ± 0.2°, 17.86° ± 0.2°, 20.84° ± 0.2°, 21.87° ± 0.2°, 26.20° ± 0.2° and 28.26° ± 0.2°.

In other embodiments, the X-ray powder diffraction pattern of crystal form A of p-toluenesulfonate salt of the compound shown in formula (I) of the present invention comprises the following diffraction peaks at 2θ angles: 6.49° ± 0.2°, 9.51° ± 0.2°, 10.04° ± 0.2°, 10.51° ± 0.2°, 10.72° ± 0.2°, 11.71° ± 0.2°, 12.65° ± 0.2°, 13.20° ± 0.2°, 14.10° ± 0.2°, 14.35° ± 0.2°, 14.94° ± 0.2°, 15.88° ± 0.2°, 16.44° ± 0.2°, 16.82° ± 0.2°, 17.21° ± 0.2°, 17.86° ± 0.2°, 18.34° ± 0.2°, 19.08° ± 0.2°, 19.57° ± 0.2°, 20.17° ± 0.2°, 20.65° ± 0.2°, 20.84° ± 0.2°, 21.26° ± 0.2°, 21.87° ± 0.2°, 22.27° ± 0.2°, 22.44° ± 0.2°, 22.84° ± 0.2°, 23.19° ± 0.2°, 23.55° ± 0.2°, 24.25° ± 0.2°, 24.81° ± 0.2°, 25.59° ± 0.2°, 26.21° ± 0.2°, 26.91° ± 0.2°, 27.38° ± 0.2°, 27.93° ± 0.2°, 28.26° ± 0.2°, 28.82° ± 0.2°, 29.16° ± 0.2°, 29.61° ± 0.2°, 30.19° ± 0.2°, 31.30° ± 0.2°, 31.90° ± 0.2°, 32.44° ± 0.2°, 33.21° ± 0.2°, 33.94° ± 0.2°, 35.32° ± 0.2°, 36.44° ± 0.2°, 37.30° ± 0.2°, 37.61° ± 0.2°, 38.59° ± 0.2°, 39.54° ± 0.2°, 42.25° ± 0.2°, 44.61° ± 0.2° and 47.32° ± 0.2°.

In some embodiments, the sulfate salt of the compound shown in formula (I) of the present invention is the crystal form A of sulfate salt, wherein the differential scanning calorimetry thermogram of the crystal form A of described sulfate salt comprises an endothermic peak of 105.07 °C ± 3 °C and 185.08 °C ± 3 °C and an exothermic peak of 233.00 °C ± 3 °C.

In some embodiments, the bisulfate salt of the compound shown in formula (I) of the present invention is the crystal form A of bisulfate salt, wherein the differential scanning calorimetry thermogram of the crystal form A of described bisulfate salt comprises an endothermic peak of 196.02°C ± 3°C and an exothermic peak of 201.85°C ± 3°C and 246.30°C ± 3°C.

In some embodiments, the phosphate salt of the compound shown in formula (I) of the present invention is the crystal form A of phosphate salt, wherein the differential scanning calorimetry thermogram of the crystal form A of described phosphate salt comprises an exothermic peak of 205.70°C ± 3°C.

In some embodiments, the p-toluenesulfonate salt of the compound shown in formula (I) of the present invention is the crystal form A of p-toluenesulfonate salt, wherein the differential scanning calorimetry thermogram of the crystal form A of described p-toluenesulfonate salt comprises an endothermic peak of 73.89°C ± 3°C and an exothermic peak of 231.68°C ± 3°C.

In some embodiments, the sulfate salt of the compound shown in formula (I) of the present invention is the crystal form A of sulfate salt, wherein, when the crystal form A of described sulfate salt is heated to 100.06 °C, the weight loss is 6.408%, and the weight loss is 3.058% when it is heated from 100.06 °C to 205.23 °C, and the error tolerance of ± 0.1% is there in the weight loss ratio.

In some embodiments, the bisulfate salt of the compound shown in formula (I) of the present invention is the crystal form A of bisulfate salt, wherein, when the crystal form A of described bisulfate salt is heated to 137.49 °C, the weight loss is 6.869%, and the weight loss is 2.873% when it is heated from 137.49 °C to 215.40 °C, and the error tolerance of ± 0.1% is there in the weight loss ratio.

In some embodiments, the phosphate salt of the compound shown in formula (I) of the present invention is the crystal form A of phosphate salt, wherein, when the crystal form A of described phosphate salt is heated to 119.39 °C, the weight loss is 3.294%, and the error tolerance of ±0.1% is there in the weight loss ratio.

In some embodiments, the p-toluenesulfonate salt of the compound shown in formula (I) of the present invention is the crystal form A of p-toluenesulfonate salt, wherein, when the crystal form A of described p-toluenesulfonate salt is heated to 150.10 °C, the weight loss is 1.709%, and the error tolerance of ± 0.1% is there in the weight loss ratio.

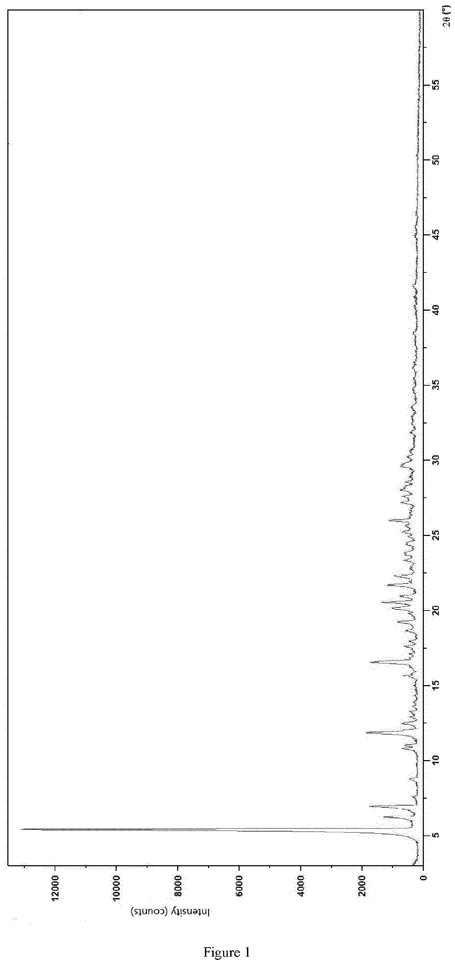

In some embodiments, the sulfate salt of the compound shown in formula (I) of the present invention is the crystal form A of sulfate salt, wherein the crystal form A of described sulfate salt has an X-ray powder diffraction pattern basically as shown in Figure 1.

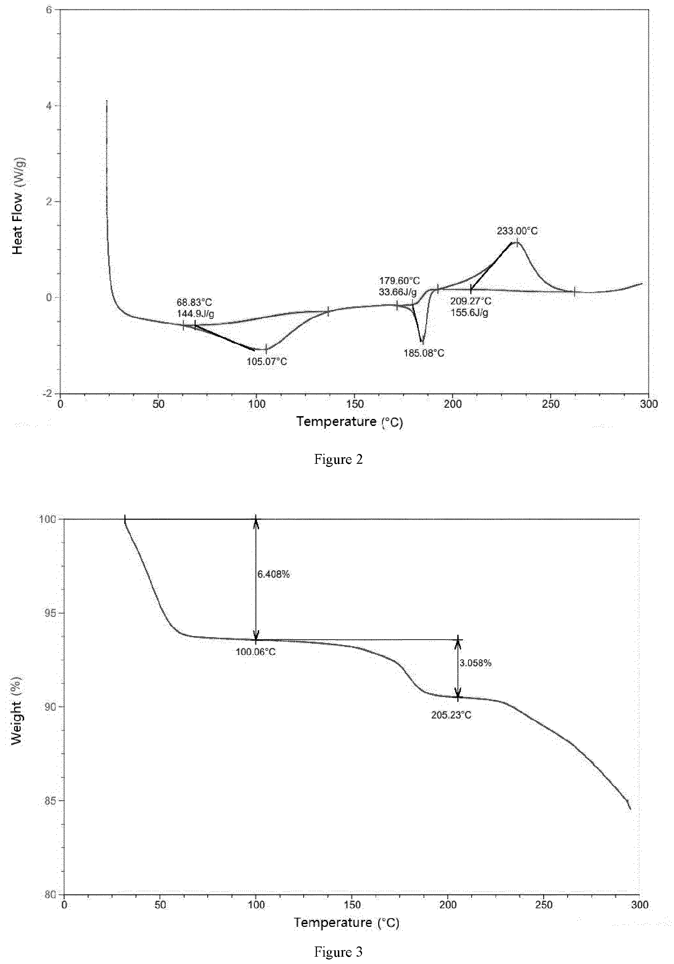

In some embodiments, the sulfate salt of the compound shown in formula (I) of the present invention is the crystal form A of sulfate salt, wherein the crystal form A of described sulfate salt has a differential scanning calorimetry thermogram basically as shown in Figure 2.

In some embodiments, the sulfate salt of the compound shown in formula (I) of the present invention is the crystal form A of sulfate salt, wherein, the crystal form A of described sulfate salt has the thermogravimetric analysis diagram that is basically as shown in Figure 3.

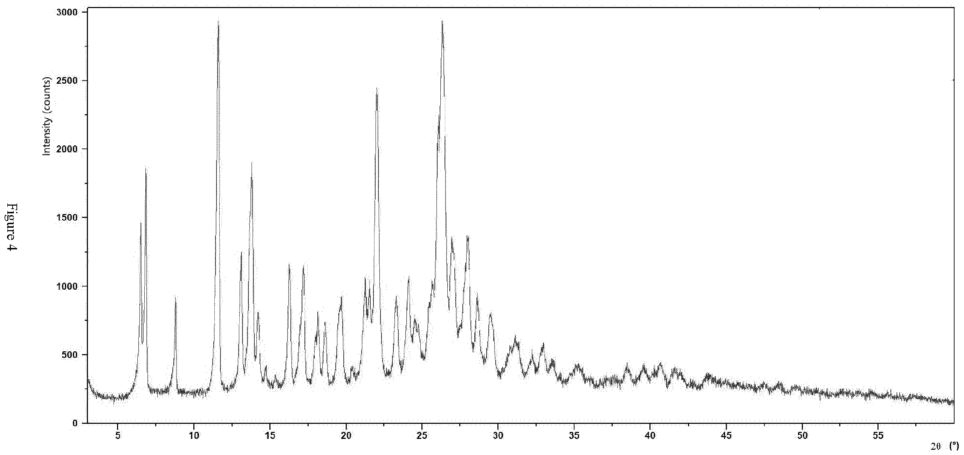

In some embodiments, the bisulfate salt of the compound shown in formula (I) of the present invention is the crystal form A of bisulfate salt, wherein the crystal form A of described bisulfate salt has an X-ray powder diffraction pattern basically as shown in Figure 4.

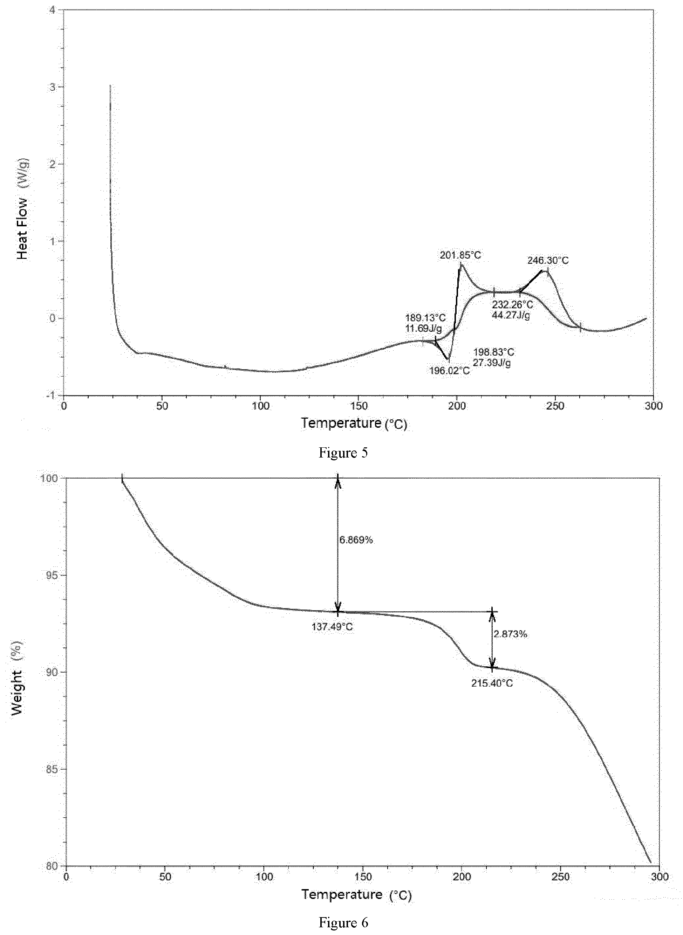

In some embodiments, the bisulfate salt of the compound shown in formula (I) of the present invention is the crystal form A of bisulfate salt, wherein the crystal form A of described bisulfate salt has a differential scanning calorimetry thermogram basically as shown in Figure 5.

In some embodiments, the bisulfate salt of the compound shown in formula (I) of the present invention is the crystal form A of bisulfate salt, wherein, the crystal form A of described bisulfate salt has the thermogravimetric analysis diagram that is basically as shown in Figure 6.

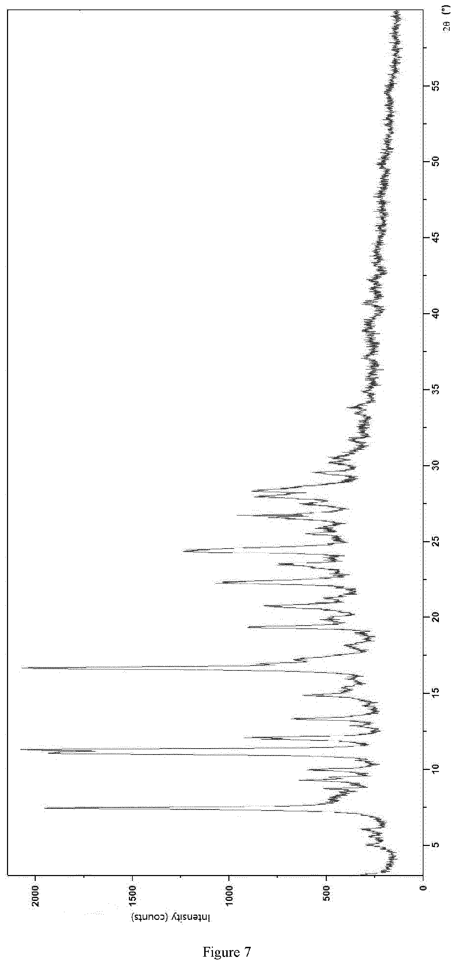

In some embodiments, the phosphate salt of the compound shown in formula (I) of the present invention is the crystal form A of phosphate salt, wherein the crystal form A of described phosphate salt has an X-ray powder diffraction pattern basically as shown in Figure 7.

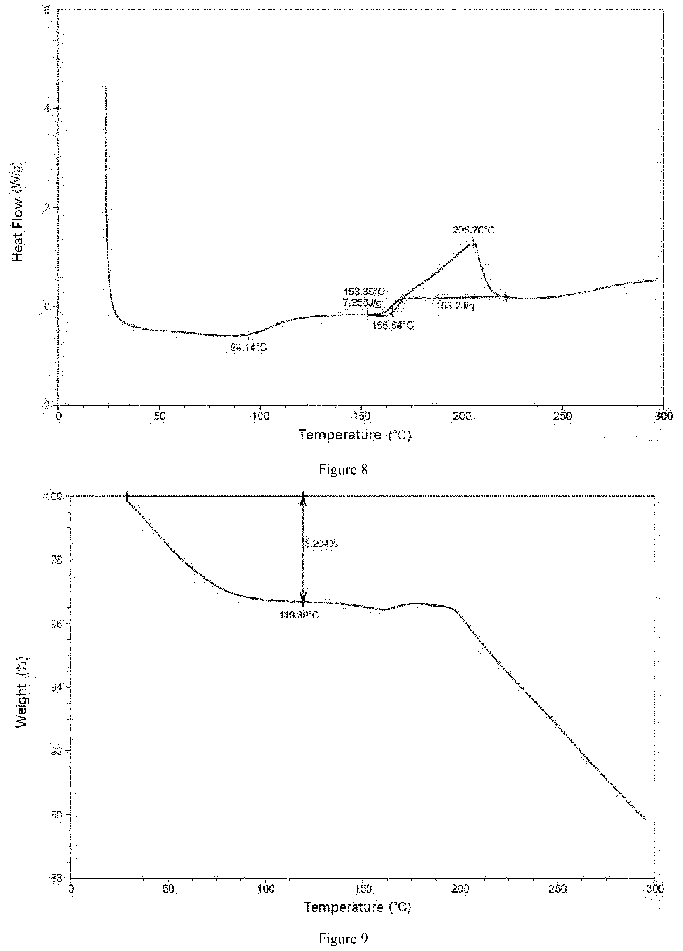

In some embodiments, the phosphate salt of the compound shown in formula (I) of the present invention is the crystal form A of phosphate salt, wherein the crystal form A of described phosphate salt has a differential scanning calorimetry thermogram basically as shown in Figure 8.

In some embodiments, the phosphate salt of the compound shown in formula (I) of the present invention is the crystal form A of phosphate salt, wherein, the crystal form A of described phosphate salt has the thermogravimetric analysis diagram that is basically as shown in Figure 9.

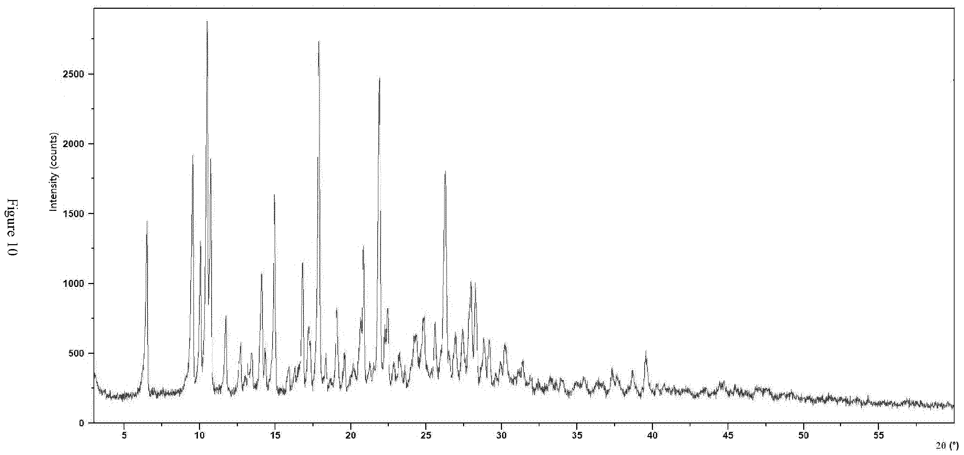

In some embodiments, the p-toluenesulfonate salt of the compound shown in formula (I) of the present invention is the crystal form A of p-toluenesulfonate salt, wherein the crystal form A of described p-toluenesulfonate salt has an X-ray powder diffraction pattern basically as shown in Figure 10.

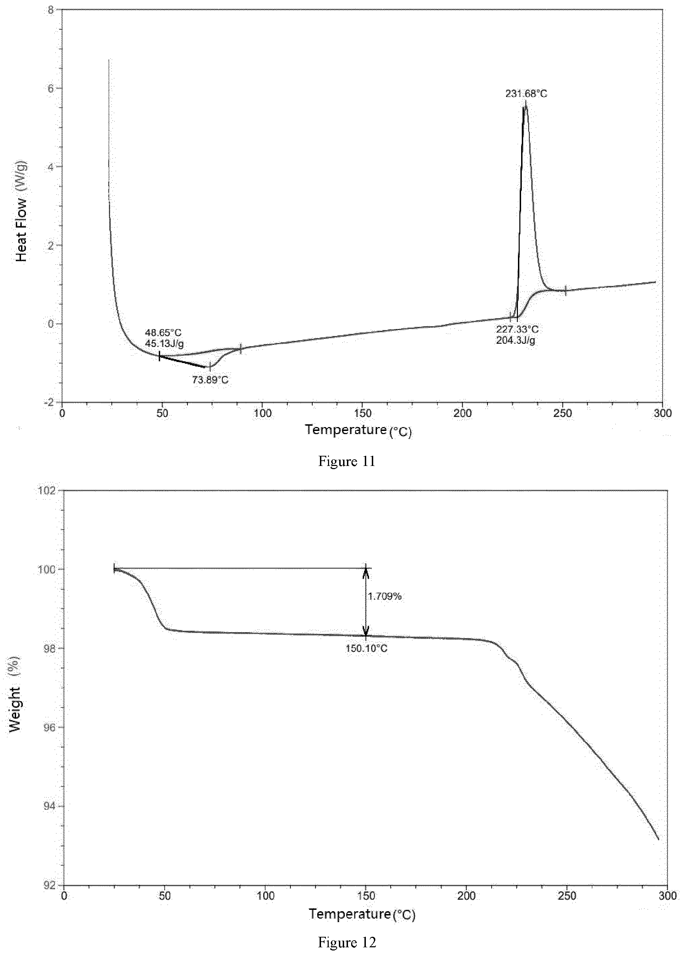

In some embodiments, the p-toluenesulfonate salt of the compound shown in formula (I) of the present invention is the crystal form A of p-toluenesulfonate salt, wherein the crystal form A of described p-toluenesulfonate salt has a differential scanning calorimetry thermogram basically as shown in Figure 11.

In some embodiments, the p-toluenesulfonate salt of the compound shown in formula (I) of the present invention is the crystal form A of p-toluenesulfonate salt, wherein, the crystal form A of described p-toluenesulfonate salt has the thermogravimetric analysis diagram that is basically as shown in Figure 12.

In other aspect, provided herein is a pharmaceutical composition comprising the salt disclosed herein and pharmaceutically acceptable carrier, excipient, diluent, adjuvant and a combination thereof.

In one aspect, provided herein is use of the salt or the pharmaceutical composition disclosed herein in the manufacture a medicament for preventing, treating or reducing diseases related to abnormal expression of PI3K kinase in patients.

In some embodiments, the disease described in the present invention is respiratory diseases, viral infections, non-viral respiratory infections, allergic diseases, autoimmune diseases, inflammatory diseases, cardiovascular diseases, hematologic malignancies, neurodegenerative diseases, pancreatitis, multi-organ failure, kidney disease, platelet aggregation, cancer, transplant rejection, lung injury or pain.

In other aspect, provided herein is use of the salt or the pharmaceutical composition disclosed herein in the manufacture a medicament for preventing, treating or reducing diseases at least partially related to abnormal expression of PI3K kinase in patients.

In some embodiments, the disease described in the present invention is asthma, chronic obstructive pulmonary disease (COPD) , viral respiratory infections, worsening of viral respiratory diseases, aspergillosis, leishmaniasis, allergic rhinitis, allergic dermatitis, rheumatoid arthritis, multiple sclerosis, inflammatory bowel disease, thrombosis, atherosclerosis, hematologic malignancies, neurodegenerative diseases, pancreatitis, multi-organ failure, nephropathy, platelet aggregation, cancer, transplant rejection, lung injury, systemic inflammatory pain, diabetic neuropathy, chronic lymphocytic leukemia (CLL), non-hodgkin's lymphoma (NHL) , hairy cell leukemia, mantle cell lymphoma, burkitt lymphoma, small lymphocytic lymphoma, follicular lymphoma, lymphoplasmaplasma cell lymphoma, extranodal marginal zone lymphoma, Hodgkin's lymphoma, Waldenström macroglobulinemia, prolymphocytic leukemia, acute lymphoblastic leukemia, myelofibrosis, mucosa-associated lymphoid tissue (MALT) lymphoma, B-cell lymphoma, thymic mediastinal large B-cell lymphoma, lymphomatoid granulomatous disease, splenic marginal zone lymphoma, primary exudative lymphoma, intravascular large B-cell lymphoma, plasma cell leukemia, extramedullary plasmacytoma, stagonic myeloma, monoclonal gammaglobulinosis (MGUS) or B-cell lymphoma.

One aspect of the present invention relates to a method of preventing, treating or ameliorating diseases associated with abnormal expression of PI3K kinase in patients, comprising administering to a patient using the salt of the invention or a pharmaceutically acceptable effective dose of the pharmaceutical composition.

In another aspect, the present invention also relates to the preparation method comprising a salt of a compound shown in formula (I).

The solvent used in the production method of the invention is not particularly restricted, and any solvent which dissolves the starting material to a degree and does not affect its properties is contained in the present invention. Additionally, many similar modifications in the art, equivalent replacements, or solvent, solvent composition and the solvent composition with different proportions which are equivalent to those described in the invention, all are deemed to be included in the present invention. The present invention gives the preferred solvent for each reaction step.

The preparation of the salt or crystal form thereof of the present invention will be described in detail in the examples section. Meanwhile, the present invention provides pharmacological test experiment (e.g., a pharmacokinetic experiment) and a wetting test of the salt or crystal form thereof. It has been proved by experiments that the salt or crystal form thereof described in the present invention has good stability and pharmacokinetic properties.

### DEFINITIONS AND GENERAL TERMINOLOGY

Unless otherwise indicated, all technical and scientific terms used in the present invention have the same meaning as commonly understood by one of ordinary skill in the art to which this invention pertains. All patents and publications referred to herein are incorporated by reference in their entirety. Although any methods and materials similar or identical to those described herein may be used in the practice or testing of the invention, but the methods, apparatus and materials described in the invention are preferred.

"Crystal form" or "crystalline form" refers to a solid having a highly regular chemical structure, including, but not limited to, mono- or multi-component crystals, and/or polymorphic compounds of compounds, solvates, hydrates, clathrates, eutectic, salt, salt solvent, salt hydrate. The crystalline form of the material can be obtained by a number of methods known in the field. Such methods include, but are not limited to, melt crystallization, melt cooling, solvent crystallization, crystallization in defined space, for example, in nanopores or capillaries, on a surface or template, for example, on a polymer, in the presence of additives such as co-crystallization counterions, removing solvent, dehydration, rapid evaporation, rapid cooling, slow cooling, vapor diffusion, sublimation, reaction crystallization, anti-solvent addition, grinding and solvent drop milling
"Solvent" refers to a substance (typically a liquid) that is capable of completely or partially dissolving another substance (typically a solid). Solvents for use in the practice of this invention include, but are not limited to, water, acetic acid, acetone, acetonitrile, benzene, chloroform, carbon tetrachloride, dichloromethane, dimethylsulfoxide, 1,4-dioxane, ethanol, ethyl acetate, butanol, t-butanol, *N*,*N*-dimethylacetamide, *N*,*N*-dimethylformamide, formamide, formic acid, heptane, hexane, isopropanol, methanol, methyl ethyl ketone, mesitylene, nitromethane, polyethylene glycol, propanol, pyridine, tetrahydrofuran, toluene, xylene, mixtures thereof, and the like.

"Anti-solvent" refers to a fluid that promotes the precipitation of a product (or product precursor) from a solvent. The anti-solvent may comprise a cold gas, or a fluid that promotes the precipitation of the fluid by chemical reaction or reduces the solubility of the product in the solvent; it may be the same liquid as the solvent but at a different temperature, or it may be a liquid different from the solvent.

"Solvate" refers to a compound that on a surface, in a lattice or having a solvent on a surface or in a lattice. The solvent can be water, acetic acid, acetone, acetonitrile, benzene, chloroform, carbon tetrachloride, dichloromethane, dimethylsulfoxide, 1,4-dioxane, ethanol, ethyl acetate, butanol, t-butanol, *N*,*N*-dimethylacetamide, *N*,*N*-dimethylformamide, formamide, formic acid, heptane, hexane, isopropanol, methanol, methyl ethyl ketone, methyl pyrrolidone, mesitylene, nitromethane, polyethylene glycol, propanol, pyridine, tetrahydrofuran, toluene, xylene, mixtures thereof, and the like. A specific example of the solvate is a hydrate in which the solvent on the surface, in the lattice or on the surface and in the lattice is water. On the surface, in the lattice or on the the surface and in the lattic of the substance, the hydrate may or may not have any solvent other than water.

Crystal form can be identified by a variety of technical means, such as X-ray powder diffraction (XRPD), infrared absorption spectroscopy (IR), melting point method, differential scanning calorimetry (DSC), thermogravimetric analysis (TGA), Nuclear magnetic resonance, Raman spectroscopy, X-ray single crystal diffraction, dissolution calorimetry, scanning electron microscopy (SEM), quantitative analysis, solubility and dissolution rate.

X-ray powder diffraction (XRPD) can detect changes in crystal form, crystallinity, crystal state and other information, is a common means for identifying crystal form. The peak position of the XRPD pattern primarily depends on the structure of the crystal form and is relatively insensitive to the experimental details, and its relative peak height depends on many factors associated with sample preparation and instrument geometry. Thus, in some embodiments, the crystalline form of the present invention is characterized by an XRPD map having certain peak positions, which is substantially as shown in the XRPD diagram provided in the drawings of the present invention at the same time, the 2θ of the XRPD pattern can be measured with an experimental error. The measurement of 2θ of the XRPD pattern may be slightly different between the different instruments and the different samples. Therefore, the value of 2θ can not be regarded as absolute. According to the condition of the instrument used in this test, the diffraction peak has an error tolerance of ± 0.2°

Differential Scanning Calorimetry (DSC) is a technique of measuring the energy difference between a sample and an inert reference (commonly used α-Al₂O₃) with temperature by continuously heating or cooling under program control The high endothermic peak of the DSC curve depends on many factors associated with sample preparation and instrument geometry, while the peak position is relatively insensitive to experimental details. Thus, in some embodiments, the crystal form of the present invention is characterized by an DSC map having certain peak positions, which is substantially as shown in the DSC diagram provided in the drawings of the present invention at the same time, the DSC pattern can be measured with an experimental error. The peak position and peak value of DSC pattern may be slightly different between the different instruments and the different samples. Therefore, the peak position or the peak value of the DSC endothermic peak can not be regarded as absolute. According to the condition of the instrument used in this test, the endothermic peak has an error tolerance of ± 3°.

Thermogravimetric analysis (TGA) is a technique for measuring the quality of a substance with temperature under the control of a program. It is suitable for examining the process of the solvent loss or the samples sublimation and decomposition. It can be presumed that the crystal contains crystal water or crystallization solvent. The quality variety of the TGA curve shown depend on a number of factors, contains the sample preparation and the instrument. The quality varity of the TGA test may be slightly different between the different instruments and between the different samples. According to the condition of the instrument used in this test, there is a ± 0.1% error tolerance for the mass change.

In the context of the present invention, the 2θ values in the X ray powder diffraction pattern are in degrees (°).

The term "substantially as shown in the figure" refers to at least 50%, or at least 60%, or at least 70%, or at least 80%, or at least 90%, or at least 95%, or at least 99% of the peaks are shown in the X-ray powder diffraction pattern or DSC pattern or Raman spectra pattern or infrared spectra pattern.

The "peak" refers to a feature that a person skilled in the art can recognize without belonging to background noise when referring to a spectrum or/and data that appears in the figure.

The present invention relates to the salt and crystal form thereof of (*S*)-2-(1-((6-amino-5-(3-methyl-1,2,4-oxadiazol-5-yl)pyrimidin-4-yl)amino)ethyl)-5-(3-hydroxypl rop-1-yn-1-yl)-3-phenylquinazolin-4(3*H*)-one, for example, crystal form A of p-toluenesulfonate salt, which exists in substantially pure crystalline form.

"Substantially pure" means that a crystalline form is substantially free of another or more crystalline forms, that means the purity of the crystalline form is at least 80%, or at least 85%, or at least 90%, or at least 93%, or at least 95%, or at least 98%, or at least 99%, or at least 99.5%, or at least 99.6%, or at least 99.7%, or at least 99.8%, or at least 99.9%, or crystal form containing other crystal form. The percentage of the other crystals in the total volume or total weight of the crystal form is less than 20%, or less than 10%, or less than 5%, or less than 3%, or less than 1%, or less than 0.5%, or less than 0.1%, or less than 0.01%.

"Substantially free" means that the percentage of one or more other crystalline forms in the total volume or total weight of the crystalline form is less than 20%, or less than 10%, or less than 5%, or less than 4%, or less than 3%, or less than 2%, or less than 1%, or less than 0.5%, or less than 0. 1%, or less than 0.01%.

XRPD pattern in a "Relative strength" (or "Relative Peak Height") means the ratio of the intensity of the other peaks to the intensity of the first strong peak when the intensity of the first strong peak in all the diffraction peaks) of the X-ray powder diffraction pattern(XRPD) is 100%.

In the context of the present invention, when used or whether or not used the word, such as "about", it means that within a given value or range of 10% or less, appropriately within 5%, especially within 1%. Or, for those of ordinary skill in the art, the term "about" means within an acceptable standard error range of the mean value. When a number with an N value is made public, any number within N +/- 1%, N +/- 2%, N +/- 3%, N +/- 5%, N +/- 7%, N +/- 8%, or N +/- 10% will be opened clearly, wherein "+/-" means plus or minus.

In the present invention, "room temperature" refers to the temperature from about 10 °C to about 40 °C. In some embodiments, "room temperature" refers to a temperature from about 20 °C to about 30 °C; in other embodiments, "room temperature" refers to 20 °C, 22.5 °C, 25 °C, 27.5 °C and so on.

### Pharmaceutical compositions, formulations, administration and uses of salts of the compounds of the present invention

The pharmaceutical composition of the present invention is characterized by salts of the compounds of formula (I), and pharmaceutically acceptable carriers, adjuvants, or excipients. The amount of salts of the compound in the pharmaceutical composition of the present invention can effectively and detectably treat or alleviate diseases associated with PI3K kinase abnormalities.

As described above, the pharmaceutically acceptable compositions disclosed herein further comprise acceptable carriers, adjuvants, or excipients, which, as used herein, includes any and all solvents, diluents, or other liquid excipient, dispersion or suspension aids, surface active agents, isotonic agents, thickening, emulsifying agents, preservatives, solid binders or lubricants, and the like, as suited to the particular dosage form desired. As described in the following: In Remington: Troy et al., Remington: The Science and Practice of Pharmacy, 21st ed., 2005, Lippincott Williams & Wilkins, Philadelphia, and Swarbrick et al., Encyclopedia- Technology, eds. 1988-1999, Marcel Dekker, New York, both of which are herein incorporated by reference in their entireties, discloses various carriers used in formulating pharmaceutically acceptable compositions and known techniques for the preparation thereof. Except insofar as any conventional carrier medium incompatible with the salt of the compounds or crystal form thereof disclosed herein, such as by producing any undesirable biological effect or otherwise interacting in a deleterious manner with any other components of the pharmaceutically acceptable composition, its use is contemplated to be within the scope of this invention.

Some non-limiting examples of materials which can serve as pharmaceutically acceptable carriers include ion exchangers; aluminium; aluminum stearate; lecithin; serum proteins such as human serum albumin; buffer substances such as phosphates; glycine; sorbic acid; potassium sorbate; partial glyceride mixtures of saturated vegetable fatty acids; water; salts or electrolytes such as protamine sulfate, disodium hydrogen phosphate, potassium hydrogen phosphate, sodium chloride, zinc salts; colloidal silica; magnesium trisilicate; polyvinyl pyrrolidone; polyacrylates; waxes; polyethylene-polyoxypropylene-block polymers; wool fat; sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil, safflower oil, sesame oil, olive oil, corn oil and soybean oil; glycols such as propylene glycol and polyethylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol; and phosphate buffer solutions; as well as other non-toxic compatible lubricants such as sodium lauryl sulfate and magnesium stearate; as well as coloring agents; releasing agents; coating agents; sweetening; flavoring; perfuming agents; preservatives and antioxidants.

The compositions disclosed herein may be capsules, tablets, pills, powders, granule and aqueous suspension or solution; it can be administered in the following ways: orally, by injection, by inhalation spray, topically, rectally, nasally, buccally, vaginally or via an implanted reservoir.

Oral administration can be given in the following forms: tablets, pills, capsules, dispersible powders, granules or suspensions, syrups, and elixirs; topical administration can be given in the following forms: ointments, gels, medicated adhesive tapes, etc.

The salts or crystal forms thereof of the present invention are preferably formulated in dosage form according to the formulation to reduce the dose and dose uniformity. The term "dosage unit type" herein refers to a physically dispersed unit in which a patient obtains the appropriate treatment. However, it should be understood that the daily general use of the salts of the compounds in formula (I) of the present invention or crystal forms thereof, or pharmaceutical compositions of the present invention will be determined by the attending physician on the basis of a reliable medical range judgment. The specific effective dose level for any particular patient or organism will depend on a number of factors including the severity of the disease and condition being treated, the activity of the salt of a particular compound or crystal form thereof, the particular composition used, the age, weight, health, sex and eating habits of the patient, time of administration, route of administration and excretion rate of the salt of the particular compound or crystal form thereof used, duration of treatment, drug use in combination or in combination with the salt of a specific compound or crystal form thereof, and other well-known factors in the field of pharmacy.

The effective dose of the active ingredient used can vary with the salt of the compound used or crystal form thereof, the mode of administration and the severity of the disease to be treated. However, usually when the salt of the compound of the present invention or crystal form thereof is given daily at a dose of about 0.25-1000 mg/kg animal body weight, satisfactory results can be obtained, preferably given in 2-4 divided doses per day, or in an extended-release form. For most large mammals, the total daily dose is about 1 to 100 mg/kg, preferably about 2 to 80 mg/kg of salts or crystal form thereof of the active compound. A dosage form suitable for oral administration containing salt or crystal form thereof of approximately 0.25-500 mg of the active compound closely mixed with a solid or liquid pharmaceutically acceptable carrier. This dose regimen can be adjusted to provide an optimal treatment response. In addition, depending on the treatment status, several divided doses may be given each day, or the dose may be reduced proportionally.

The salt of the compound of the present invention or crystal form thereof, the pharmaceutical composition of the present invention can be used to inhibit the activity of PI3K kinase, thereby regulating the stability and/or activity of PI3K kinase and activating the expression of PI3K regulatory genes. The salt of compound or crystal form thereof or the the pharmaceutical composition can be used in the treatment, pre-treatment or delay of the onset or development of PI3K kinase-related conditions, including but not limited to hematologic malignancies.

Specifically, the salt of the compound of the present invention or crystal form thereof may be used to inhibit the activity of PI3K kinase. The salt of the compound or crystal form thereof may be administered to prevent, pre-treatment, or treat conditions associated with abnormal expression of PI3K kinase, including, for example, asthma, chronic obstructive pulmonary disease (COPD), viral respiratory infections, worsening of viral respiratory diseases, aspergillosis, leishmaniasis, allergic rhinitis, allergic dermatitis, rheumatoid arthritis, multiple sclerosis, inflammatory bowel disease, thrombosis, atherosclerosis, hematologic malignancies, neurodegenerative diseases, pancreatitis, multi-organ failure, nephropathy, platelet aggregation, cancer, sperm motility, transplant rejection, lung injury, systemic inflammatory pain, diabetic neuropathy, chronic lymphocytic leukemia (CLL), non-hodgkin's lymphoma (NHL) , hairy cell leukemia, mantle cell lymphoma, burkitt lymphoma, small lymphocytic lymphoma, follicular lymphoma, lymphoplasmaplasma cell lymphoma, extranodal marginal zone lymphoma, Hodgkin's lymphoma, Waldenström macroglobulinemia, prolymphocytic leukemia, acute lymphoblastic leukemia, myelofibrosis, mucosa-associated lymphoid tissue (MALT) lymphoma, B-cell lymphoma, mediastinal (thymic) large B-cell lymphoma, lymphomatoid granulomatous disease, splenic marginal zone lymphoma, primary exudative lymphoma, intravascular large B-cell lymphoma, plasma cell leukemia, extramedullary plasmacytoma, stagonic myeloma ((also known as asymptomatic myeloma)), monoclonal gammaglobulinosis of undetermined significance(MGUS) or B-cell lymphoma.

### Description of the drawings

Figure 1 is an X-ray powder diffraction (XRPD) plot of the crystal form A of sulfate salt of the compound shown in formula (I).
Figure 2 is a differential scanning calorimetry (DSC) plot of the crystal form A of sulfate salt of the compound shown in formula (I).
Figure 3 is a thermogravimetric (TGA) analysis of the crystal form A of sulfate salt of the compound shown in formula (I).
Figure 4 is an X-ray powder diffraction (XRPD) plot of the crystal form A of bisulfate salt of the compound shown in formula (I).
Figure 5 is a differential scanning calorimetry (DSC) plot of the crystal form A of bisulfate salt of the compound shown in formula (I).
Figure 6 is a thermogravimetric (TGA) analysis of the crystal form A of bisulfate salt of the compound shown in formula (I).
Figure 7 is an X-ray powder diffraction (XRPD) plot of the crystal form A of phosphate salt of the compound shown in formula (I).
Figure 8 is a differential scanning calorimetry (DSC) plot of the crystal form A of phosphate salt of the compound shown in formula (I).
Figure 9 is a thermogravimetric (TGA) analysis of the crystal form A of phosphate salt of the compound shown in formula (I).
Figure 10 is an X-ray powder diffraction (XRPD) plot of the crystal form A of p-toluenesulfonate salt of the compound shown in formula (I).
Figure 11 is a differential scanning calorimetry (DSC) plot of the crystal form A of p-toluenesulfonate salt of the compound shown in formula (I).
Figure 12 is a thermogravimetric (TGA) analysis of the crystal form A of p-toluenesulfonate salt of the compound shown in formula (I).
Figure 13 is an X-ray powder diffraction (XRPD) plot of the free base crystal form 2 of the compound shown in formula (I).

### EXAMPLES

The invention will now be further described by way of example without limiting the invention to the scope of the invention.

The X-ray powder diffraction analysis method used in the present invention was an Empyrean diffractometer, and an X-ray powder diffraction pattern was obtained using Cu-Kα radiation (45 KV, 40 mA). The powdery sample was prepared as a thin layer on a monocrystalline silicon sample rack and placed on a rotating sample stage, analyzed at a rate of 0.0168° steps in the range of 3 °-40 °. Use Data Collector software to collect data, HighScore Plus software to process data, Data Viewer software to read data.

The differential scanning calorimetry (DSC) analysis method used in the present invention is a differential scanning calorimeter using a TA Q2000 module with a thermal analysis controller. Data were collected and analyzed using TA Instruments Thermal Solutions software. Approximately 1-5 mg of the sample was accurately weighed into a specially crafted aluminum crucible with a lid and analyzed from room temperature to about 300 °C using a linear heating device at 10 °C/min. During use, the DSC chamber was purged with dry nitrogen.

The thermogravimetric (TGA) analysis method used in the present invention is a thermogravimetric analysis using a TA Q500 module with a thermal analysis controller. Data were collected and analyzed using TA Instruments Thermal Solutions software. Approximately 10 mg of the sample was accurately weighed into a platinum sample tray and analyzed from room temperature to about 300 °C using a linear heating device at 10 °C/min. During use, the TGA chamber was purged with dry nitrogen.

The compound (*S*)-2-(1-((6-amino-5-(3-methyl-1,2,4-oxadiazol-5-yl)pyrimidin-4-yl)amino)ethyl)-5-(3-hydroxyp rop-1-yn-1-yl)-3-phenylquinazolin-4(3*H*)-one shown in formula (I) is obtained by reference to the synthesis method in Example 34 of the international application WO2019143874 A1.

### Example

### Example 1 the crystal form A of sulfate salt of the compound shown in formula (I)

### 1. Preparation of crystal form A of sulfate salt

A water (0.3 mL) solution of sulfuric acid (0.11 g, 1.14 mmol) was added to the methanol (4.0 mL) solution of (*S*)-2-(1-((6-amino-5-(3-methyl-1,2,4-oxadiazol-5-yl)pyrimidin-4-yl)amino)ethyl)-5-(3-hydroxyp rop-1-yn-1-yl)-3-phenylquinazolin-4(3*H*)-one (0.50 g, 1.01 mmol, prepared with reference to Example 5), and the sulfuric acid weighing flask was then rinsed with water (0.2 mL) and added to the system. The system was heated to reflux and stirred for 20 min, methanol (6.0 mL) was added, and the reflux stirring was continued for 4 h, and the system could not be dissolved. The mixture was cooled to room temperature, filtered, and the filter cake was blasted and dried at 60°C for 12 h, and the crystal form A of sulfate salt was obtained as a white solid (0.46 g, yield 77%).

### 2. Identification of crystal form A of sulfate salt

(1) Analysis and identification by Empyrean X-ray powder diffraction (XRPD) using Cu-Kα radiation with the following characteristic peaks at an angle of 2θ: 5.37°, 6.20°, 6.90°, 8.74°, 10.77°, 10.99°, 11.82°, 12.45°, 15.61°, 16.51°, 16.60°, 17.08°, 17.57°, 17.91°, 18.62°, 19.18°, 20.10°, 20.50°, 20.91°, 21.64°, 22.24°, 23.31°, 23.70°, 23.83°, 24.41°, 24.87°, 25.18°, 25.59°, 25.95°, 27.15°, 27.55°, 27.98°, 28.19°, 28.51°, 29.63°, 30.19°, 31.83°, 36.11° and 41.55°. There is an error tolerance of ± 0.2 °.
(2) Analysis and identification by TA Q2000 Differential Scanning Calorimetry: the scanning speed is 10 °C/min and and the resulting DSC curve is basically as shown in Figure 2, comprising an endothermic peaks of 105.07 °C and 185.08 °C and exothermic peak of 233.00 °C. There is an error tolerance of ± 3 °C.
(3) Analysis and identification by TA Q500 thermogravimetric Analysis (TGA): the TGA curve obtained at a heating rate of 10°C/min is basically as shown in Figure 3, which comprises 6.408% weight loss when heated to 100.06°C, and 3.058% weight loss when heated from 100.06°C to 205.23°C. There is an error tolerance of ± 0.1%.

### Example 2 the crystal form A of bisulfate salt of the compound shown in formula (I)

### 1. Preparation of crystal form A of bisulfate salt

A water (0.3 mL) solution of sulfuric acid (0.11 g, 1.12 mmol) was added to the acetonitrile (4.0 mL) solution of (*S*)-2-(1-((6-amino-5-(3-methyl-1,2,4-oxadiazol-5-yl)pyrimidin-4-yl)amino)ethyl)-5-(3-hydroxyp rop-1-yn-1-yl)-3-phenylquinazolin-4(3*H*)-one (0.50 g, 1.01 mmol, prepared with reference to Example 5), and the sulfuric acid weighing flask was then rinsed with water (0.2 mL) and added to the system. The system was heated to reflux and stirred for 20 min, acetonitrile (6.0 mL) was added, and the reflux stirring was continued for 4 h, and the system was dissolved. The system was cooled to room temperature, filtered, rinsed with acetonitrile (2.0 mL), and the filter cake was blasted and dried at 60°C for 12 h, and the crystal form A of bisulfate salt was obtained as a light yellow solid (0.60 g, yield 100%).

### 2. Identification of crystal form A of bisulfate salt

(1) Analysis and identification by Empyrean X-ray powder diffraction (XRPD) using Cu-Kα radiation with the following characteristic peaks at an angle of 2θ: 6.49°, 6.81°, 8.75°, 11.54°, 13.06°, 13.64°, 13.79°, 14.19°, 14.70°, 16.24°, 17.11°, 18.10°, 18.59°, 19.48°, 19.68°, 21.20°, 21.52°, 21.99°, 23.25°, 24.05°, 24.57°, 25.57°, 25.99°, 26.32°, 26.90°27.08°, 27.91°, 28.61°, 29.48°, 31.02°, 32.88°, 33.53°, 35.20°, 38.45°, 39.52°, 40.58°, 41.72° and 43.81°. There is an error tolerance of ± 0.2 °.
(2) Analysis and identification by TA Q2000 Differential Scanning Calorimetry: the scanning speed is 10 °C/min and and the resulting DSC curve is basically as shown in Figure 5, comprising an endothermic peak of 196.02 °C and exothermic peaks of 201.85 °C and 246.30 °C . There is an error tolerance of ± 3 °C.
(3) Analysis and identification by TA Q500 thermogravimetric Analysis (TGA): the TGA curve obtained at a heating rate of 10°C/min is basically as shown in Figure 6, which comprises 6.869% weight loss when heated to 137.49°C, and 2.873% weight loss when heated from 137.49°C to 215.40°C. There is an error tolerance of ± 0.1%.

### Example 3 the crystal form A of phosphate salt of the compound shown in formula (I)

### 1. Preparation of crystal form A of phosphate salt

A water (0.3 mL) solution of phosphoric acid (0.11 g, 1.16 mmol) was added to the acetonitrile (4.0 mL) solution of (*S*)-2-(1-((6-amino-5-(3-methyl-1,2,4-oxadiazol-5-yl)pyrimidin-4-yl)amino)ethyl)-5-(3-hydroxyp rop-1-yn-1-yl)-3-phenylquinazolin-4(3*H*)-one (0.50 g, 1.02 mmol, prepared with reference to Example 5), and the phosphoric acid weighing flask was then rinsed with water (0.2 mL) and added to the system. The system was heated to reflux and stirred for 20 min, acetonitrile (6.0 mL) was added, and the reflux stirring was continued for 4 h, and the system could not be dissolved. The mixture was cooled to room temperature, filtered, rinsed with acetonitrile (2.0 mL), and the filter cake was blasted and dried at 60°C for 12 h, and the crystal form A of phosphate salt was obtained as a white solid (0.57 g, 95% yield).

### 2. Identification of crystal form A of phosphate salt

(1) Analysis and identification by Empyrean X-ray powder diffraction (XRPD) using Cu-Kα radiation with the following characteristic peaks at an angle of 2θ: 5.02°, 5.95°, 7.41°, 8.15°, 9.27°, 9.93°, 11.06°, 11.27°, 12.02°, 12.82°, 13.30°, 14.87°, 16.62°, 17.09°, 18.11°, 19.32°, 19.79°, 20.69°, 21.22°, 22.27°, 23.41°, 24.38°, 25.47°, 26.58°, 27.43°, 27.87°, 28.32°, 29.45°, 30.34°, 31.62° and 33.79°. There is an error tolerance of ± 0.2 °.
(2) Analysis and identification by TA Q2000 Differential Scanning Calorimetry: the scanning speed is 10 °C/min and and the resulting DSC curve is basically as shown in Figure 8, comprising an exothermic peak of 205.70 °C. There is an error tolerance of ± 3 °C.
(3) Analysis and identification by TA Q500 thermogravimetric Analysis (TGA): the TGA curve obtained at a heating rate of 10°C/min is basically as shown in Figure 9, which comprises 3.294% weight loss when heated to 119.39°C. There is an error tolerance of ± 0.1%.

### Example 4 the crystal form A of p-toluenesulfonate salt of the compound shown in formula (I)

### 1. Preparation of crystal form A of p-toluenesulfonate salt

A water (60 mL) solution of p-toluenesulfonic acid monohydrate (16.13 g, 84.80 mmol) was added to a suspension solution of (*S*)-2-(1-((6-amino-5-(3-methyl-1,2,4-oxadiazol-5-yl)pyrimidin-4-yl)amino)ethyl)-5-(3-hydroxyp rop-1-yn-1-yl)-3-phenylquinazolin-4(3*H*)-one (38.00 g, 76.85 mmol, prepared with reference to Example 5), ethanol (230 mL) and water (150 mL), and the bottle containing the acid solution was then rinsed with water (20 mL) and added to the system. The system was heated to reflux and stirred for 2 h, then reduced to room temperature and stirred for 30 min, water (690 mL) was added, stirring was continued for 1 h, filtered, and the reaction bottle was washed with ethanol/water (v/v = 1/4, 50 mL), and the washing solution was filtered together. The filter cake was blasted and dried at 60°C overnight to obtain a light yellow solid (45.8 g, yield 89.4%).

### 2. Identification of crystal form A of p-toluenesulfonate salt

(1) Analysis and identification by Empyrean X-ray powder diffraction (XRPD) using Cu-Kα radiation with the following characteristic peaks at an angle of 2θ: 6.49°, 9.51°, 10.04°, 10.51°, 10.72°, 11.71°, 12.65°, 13.20°, 14.10°, 14.35°, 14.94°, 15.88°, 16.44°, 16.82°, 17.21°, 17.86°, 18.34°, 19.08°, 19.57°, 20.17°, 20.65°, 20.84°, 21.26°, 21.87°, 22.27°, 22.44°, 22.84°, 23.19°, 23.55°, 24.25°, 24.81°, 25.59°, 26.21°, 26.91°, 27.38°, 27.93°, 28.26°, 28.82°, 29.16°, 29.61°, 30.19°, 31.30°, 31.90°, 32.44°, 33.21°, 33.94°, 35.32°, 36.44°, 37.30°, 37.61°, 38.59°, 39.54°, 42.25°, 44.61° and 47.32°. There is an error tolerance of ± 0.2 °.
(2) Analysis and identification by TA Q2000 Differential Scanning Calorimetry: the scanning speed is 10 °C/min and and the resulting DSC curve is basically as shown in Figure 11, comprising an endothermic peak of 73.89 °C and exothermic peaks of 231.68 °C. There is an error tolerance of ± 3 °C.
(3) Analysis and identification by TA Q500 thermogravimetric Analysis (TGA): the TGA curve obtained at a heating rate of 10°C/min is basically as shown in Figure 12, which comprises 1.709% weight loss when heated to 150.10°C. There is an error tolerance of ± 0.1%.

### Example 5 free base crystal form 2 of the compound shown in formula (I)

### 1. Preparation of free base crystal form 2 shown in formula (I)

The free base crystal form 2 was obtained by reference to the synthesis method of embodiment 34 in the international application WO2019143874 A1.

### 2. Identification of free base crystal form 2 shown in formula (I)

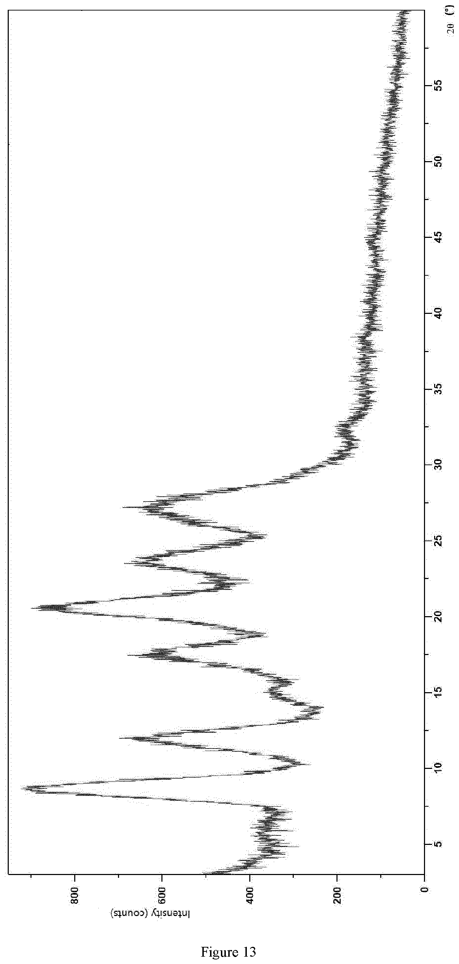

Analysis and identification by Empyrean X-ray powder diffraction (XRPD) using Cu-Kα radiation. In this example, the X-ray powder diffraction pattern of free base crystal form 2 was prepared by the method, which was basically shown in Figure 13.

### Example 6 The pharmacokinetics test of the salt of the present invention

### Experimental method for pharmacokinetic testing of salt and free base crystal form 2 in Beagle dog of the present invention

The salt of the compound shown in formula (I) and the free base crystal form 2 of the compound shown in formula (I) of the present invention were respectively filled with capsules for oral administration.

Male Beagle dogs of 8-12 kg were divided into 2 groups, 3 dogs in each group, and capsules containing test samples were orally administered at a dose of 10 mg/kg, and blood was collected at time points of 0.25, 0.5, 1.0, 2.0, 4.0, 6.0, 8.0 and 24 h. Standard curve was plotted based on concentrations of the samples in a suitable range, the concentration of the test sample in the plasma sample was measured and quantified by AB SCIEX API4000-type LC-MS / MS at MRM mode. Pharmacokinetic parameters were calculated according to drug concentration -time curve using a noncompartmental method by WinNonLin 6.3 software. Results were as shown in table 1.

**Table 1 Pharmacokinetic experimental results data of salt and free base crystal form 2 of the present invention in Beagle dogs**

| Test sample | dosage (mg/kg) | AUCₗₐₛₜ (h*ng/ml) | Cₘₐₓ (ng/ml) | Tₘₐₓ (h) |
|---|---|---|---|---|
| crystal form A of p-toluenesulfonate salt | 10 | 84200 ± 20000 | 8400 ± 1800 | 3 ± 1.7 |
| Free base crystal form 2 | 10 | 26700 ± 6300 | 2480 ± 600 | 2.36 ± 1.2 |

Conclusion: The salt described in the present invention has good pharmacokinetic properties in Beagle dogs, especially the exposure of crystal form A of p-toluenesulfonate salt in Beagle dogs is higher, and the blood concentration is also higher, and it has better pharmacokinetic properties.

### Experimental method for pharmacokinetic testing of salt and free base crystal form 2 in SD rat of the present invention

The salt of the compound shown in formula (I) and the free base crystal form 2 of the compound shown in formula (I) of the present invention were respectively prepared into a suspension using PEG400, and the male SD rats were given 200 mg/kg test samples orally by gavage, and 3 animals in each group were given. Blood was collected at time points 0.25, 0.5, 1.0, 2.0, 5.0, 7.0 and 24 h after administration. Standard curve was plotted based on concentrations of the samples in a suitable range, the concentration of the test sample in the plasma sample was measured and quantified by AB SCIEX API4000-type LC-MS / MS at MRM mode. Pharmacokinetic parameters were calculated according to drug concentration -time curve using a noncompartmental by WinNonLin 6.3 software. Results were as shown in table 2.

**Table 2 Pharmacokinetic experimental results data of salt and free base crystal form 2 of the present invention in male SD rats**

| Test sample | dosage (mg/kg) | Tₘₐₓ (h) | Cₘₐₓ (ng/ml) | AUCₗₐₛₜ (h*ng/ml) |
|---|---|---|---|---|
| crystal form A of p-toluenesulfonate salt | 200 | 1.33 ± 0.58 | 6810 ± 1700 | 55200 ± 8700 |
| crystal form A of Sulfate salt | 200 | 1.17 ± 0.764 | 3310 ± 993 | 35300 ± 8920 |
| crystal form A of bisulfate salt | 200 | 1.50 ± 0.866 | 4080 ± 375 | 40600 ± 183 |
| crystal form A of phosphate salt | 200 | 1.50 ± 0.866 | 3950 ± 1500 | 37800 ± 6550 |
| Free base crystal form 2 | 200 | 1.67 ± 0.58 | 5800 ± 850 | 39800 ± 7600 |

### Conclusion:

As can be seen from Table 2, the salt of the present invention is exposed to a large amount in SD rats and has good pharmacokinetic properties.

### Example 7 The stability test of the salt of the present invention

### Test method

High-temperature test: The salt of the compound shown in formula (I) of the present invention was taken an appropriate amount and put into a flat weighing flask, divided into ≤ 3 mm thick thin layer. The samples were placed at 60 °C and 40 °C for 30 days. The samples were sampled at day 5, day 10 and day 30, and the sample color was observed. The purity of the sample was determined by HPLC.

High-humidity test: The salt of the compound shown in formula (I) of the present invention was taken an appropriate amount and put into a flat weighing flask, divided into ≤ 3 mm thick thin layer. The samples were placed at 25 °C, RH 90% ± 5% and 25 °C, RH 75% ± 5% for 30 days. The samples were sampled at day 5, day 10 and day 30, and the sample color was observed. The purity of the sample was determined by HPLC.

Illumination test: The salt of the compound shown in formula (I) of the present invention was taken an appropriate amount and put into a flat weighing flask, divided into ≤ 3 mm thick thin layer. Samples were placed in an open light box (with UV) at 4500 ± 500 lx and UV ≥ 0.7 w/m² for 30 days. The samples were sampled at day 5, day10 and day 30, and the sample color was observed. The purity of the sample was determined by HPLC.

Long-term stability test: The samples were inspected under the conditions of single-layer PE inner packaging, aluminum foil bag outer packaging, built-in KD-20 oxygen absorber, and vacuum nitrogen filling and heat sealing packaging, low temperature 5 °C ± 3 °C long-term test conditions, and the sample color was observed. The purity of the sample was determined by HPLC.

Results were as shown in table 3.

**Table 3 Experimental results of stability of crystal form A of p-toluenesulfonate salt described in the present invention - high temperature, high humidity and long-term stability**

| Condition | | High temperature (40°C) | | | High temperature (60°C) | | |
|---|---|---|---|---|---|---|---|
| Project | 0 day | 5 days | 10 days | 30 days | 5 days | 10 days | 30 days |
| Outward | Light yellow powder | Light yellow powder | Light yellow powder | Light yellow powder | Light yellow powder | Light yellow powder | Light yellow powder |
| Purity/% | 99.78 | 99.74 | 99.78 | 99.69 | 99.61 | 99.44 | 99.1 |

| Condition | | High humidity RH90% | | | High humidity RH75% | | |
|---|---|---|---|---|---|---|---|
| Project | 0 day | 5 days | 10 days | 30 days | 5 days | 10 days | 30 days |
| Outward | Light yellow powder | Light yellow powder | Light yellow powder | Light yellow powder | Light yellow powder | Light yellow powder | Light yellow powder |
| Purity/% | 99.78 | 99.75 | 99.78 | 99.81 | 99.69 | 99.69 | 99.65 |

| | Long-term stability test | | | | | | |
|---|---|---|---|---|---|---|---|
| Outward | 0 day | 3 months | 6 months | 9 months | 12 months | 18 months | 24 months |
| Purity/% | 99.27 | 99.26 | 99.23 | 99.26 | 99.27 | 99.24 | 99.24 |

It can be seen from the experimental results that the salt of the present invention is stable under the conditions of high temperature and high humidity, especially the appearance and purity of the crystal form A of p-toluenesulfonate salt described in the present invention are not significantly changed.

Under the condition of long-term stability test, there is no obvious change in the appearance and purity of crystal form A of p-toluenesulfonate salt of the present invention.

In summary, the stability of crystal form A of p-toluenesulfonate salt of the present invention is good and is suitable for pharmaceutical use.

The foregoing description is merely a basic illustration of the present invention and any equivalent transformation made in accordance with the technical solution of the present invention is intended to be within the scope of the present invention.

Reference throughout this specification to "an embodiment", "some embodiments", "one embodiment", "another example", "an example", "a specific examples" or "some examples" means that a particular feature, structure, material, or characteristic described in connection with the embodiment or example is included in at least one embodiment or example of the present disclosure. Thus, the appearances of the phrases such as "in some embodiments", "in one embodiment", "in an embodiment", "in another example", in an example", "in a specific examples" or "in some examples" in various places throughout this specification are not necessarily referring to the same embodiment or example of the present disclosure. Furthermore, the particular features, structures, materials or characteristics may be combined in any suitable manner in one or more embodiments or examples. In addition, those skilled in the art can integrate and combine different embodiments, examples or the features of them as long as they are not contradictory to one another.

Although explanatory embodiments have been shown and described, it would be appreciated by those skilled in the art that the above embodiments cannot be construed to limit the present disclosure, and changes, alternatives, and modifications can be made in the embodiments without departing from spirit, principles and scope of the present disclosure.

## Claims

1. A salt of a compound as shown in formular (1), wherein the salt is a sulfate salt, bisulfate salt, phosphate salt or p-toluenesulfonate salt,

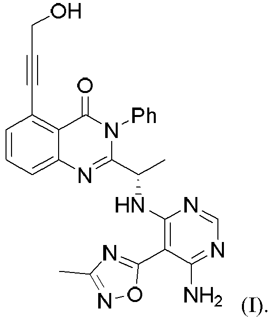

2. The salt according to claim 1, wherein the sulfate salt is the crystal form A of the sulfate salt, which is **characterized by** an X-ray powder diffraction pattern comprising peaks expressed as 2θ at 5.37° ± 0.2°, 6.90° ± 0.2°, 11.82° ± 0.2°, 16.51° ± 0.2°, 20.50° ± 0.2° and 25.95° ± 0.2°;
wherein the bisulfate salt is the crystal form A of the bisulfate salt, which is **characterized by** an X-ray powder diffraction pattern comprising peaks expressed as 2θ at 6.49° ± 0.2°, 6.81° ± 0.2°, 11.54° ± 0.2°, 13.79° ± 0.2°, 21.99° ± 0.2°, 25.99° ± 0.2° and 26.32° ± 0.2°;
wherein the phosphate salt is the crystal form A of the phosphate salt, which is **characterized by** an X-ray powder diffraction pattern comprising peaks expressed as 2θ at 7.41° ± 0.2°, 11.06° ± 0.2°, 11.27° ± 0.2°, 16.62° ± 0.2°, 19.32° ± 0.2°, 22.27° ± 0.2° and 24.38° ± 0.2°; or
wherein the p-toluenesulfonate salt is the crystal form A of the p-toluenesulfonate salt, which is **characterized by** an X-ray powder diffraction pattern comprising peaks expressed as 2θ at 6.49° ± 0.2°, 10.04° ± 0.2°, 11.71° ± 0.2°, 14.10° ± 0.2°, 17.86° ± 0.2°, 20.84° ± 0.2° and 21.87° ± 0.2°.

3. The salt according to claim 1 or 2, wherein the sulfate salt is the crystal form A of the sulfate salt, which is **characterized by** an X-ray powder diffraction pattern comprising peaks expressed as 2θ at 5.37° ± 0.2°, 6.20° ± 0.2°, 6.90° ± 0.2°, 11.82° ± 0.2°, 16.51° ± 0.2°, 16.60° ± 0.2°, 19.18° ± 0.2°, 20.10° ± 0.2°, 20.50° ± 0.2°, 21.64° ± 0.2°, 25.95° ± 0.2° and 29.63° ± 0.2°;
wherein the bisulfate salt is the crystal form A of the bisulfate salt, which is **characterized by** an X-ray powder diffraction pattern comprising peaks expressed as 2θ at 6.49° ± 0.2°, 6.81° ± 0.2°, 8.75° ± 0.2°, 11.54° ± 0.2°, 13.06° ± 0.2°, 13.64° ± 0.2°, 13.79° ± 0.2°, 16.24° ± 0.2°, 17.11° ± 0.2°, 21.99° ± 0.2°, 25.99° ± 0.2°, 26.32° ± 0.2°, 26.90° ± 0.2° and 27.91° ± 0.2°;
wherein the phosphate salt is the crystal form A of the phosphate salt, which is **characterized by** an X-ray powder diffraction pattern comprising peaks expressed as 2θ at 7.41° ± 0.2°, 9.27° ± 0.2°, 11.06° ± 0.2°, 11.27° ± 0.2°, 12.02° ± 0.2°, 13.30° ± 0.2°, 16.62° ± 0.2°, 19.32° ± 0.2°, 20.69° ± 0.2°, 22.27° ± 0.2°, 24.38° ± 0.2°, 26.58° ± 0.2°, 27.87° ± 0.2° and 28.32° ± 0.2°; or
wherein the p-toluenesulfonate salt is the crystal form A of the p-toluenesulfonate salt, which is **characterized by** an X-ray powder diffraction pattern comprising peaks expressed as 2θ at 6.49° ± 0.2°, 9.51° ± 0.2°, 10.04° ± 0.2°, 10.51° ± 0.2°, 10.72° ± 0.2°, 11.71° ± 0.2°, 14.10° ± 0.2°, 14.94° ± 0.2°, 16.82° ± 0.2°, 17.86° ± 0.2°, 20.84° ± 0.2°, 21.87° ± 0.2°, 26.20° ± 0.2° and 28.26° ± 0.2°.

4. The salt according to any one of claims 1 to 3, wherein the sulfate salt is the crystal form A of the sulfate salt, which is **characterized by** an X-ray powder diffraction pattern comprising peaks expressed as 2θ at 5.37° ± 0.2°, 6.20° ± 0.2°, 6.90° ± 0.2°, 8.74° ± 0.2°, 10.77° ± 0.2°, 10.99° ± 0.2°, 11.82° ± 0.2°, 12.45° ± 0.2°, 15.61° ± 0.2°, 16.51° ± 0.2°, 16.60° ± 0.2°, 17.08° ± 0.2°, 17.57° ± 0.2°, 17.91° ± 0.2°, 18.62° ± 0.2°, 19.18° ± 0.2°, 20.10° ± 0.2°, 20.50° ± 0.2°, 20.91° ± 0.2°, 21.64° ± 0.2°, 22.24° ± 0.2°, 23.31° ± 0.2°, 23.70° ± 0.2°, 23.83° ± 0.2°, 24.41° ± 0.2°, 24.87° ± 0.2°, 25.18° ± 0.2°, 25.59° ± 0.2°, 25.95° ± 0.2°, 27.15° ± 0.2°, 27.55° ± 0.2°, 27.98° ± 0.2°, 28.19° ± 0.2°, 28.51° ± 0.2°, 29.63° ± 0.2°, 30.19° ± 0.2° , 31.83° ± 0.2°, 36.11° ± 0.2° and 41.55° ± 0.2°
wherein the bisulfate salt is the crystal form A of the bisulfate salt, which is **characterized by** an X-ray powder diffraction pattern comprising peaks expressed as 2θ at 6.49° ± 0.2°, 6.81° ± 0.2°, 8.75° ± 0.2°, 11.54° ± 0.2°, 13.06° ± 0.2°, 13.64° ± 0.2°, 13.79° ± 0.2°, 14.19° ± 0.2°, 14.70° ± 0.2°, 16.24° ± 0.2°, 17.11° ± 0.2°, 18.10° ± 0.2°, 18.59° ± 0.2°, 19.48° ± 0.2°, 19.68° ± 0.2°, 21.20° ± 0.2°, 21.52° ± 0.2°, 21.99° ± 0.2°, 23.25° ± 0.2°, 24.05° ± 0.2°, 24.57° ± 0.2°, 25.57° ± 0.2°, 25.99° ± 0.2°, 26.32° ± 0.2°, 26.90° ± 0.2°, 27.08° ± 0.2°, 27.91° ± 0.2°, 28.61° ± 0.2°, 29.48° ± 0.2°, 31.02° ± 0.2°, 32.88° ± 0.2°, 33.53° ± 0.2°, 35.20° ± 0.2°, 38.45° ± 0.2°, 39.52° ± 0.2°, 40.58° ± 0.2°, 41.72° ± 0.2° and 43.81° ± 0.2°;
wherein the phosphate salt is the crystal form A of the phosphate salt, which is **characterized by** an X-ray powder diffraction pattern comprising peaks expressed as 2θ at 5.02° ± 0.2°, 5.95° ± 0.2°, 7.41° ± 0.2°, 8.15° ± 0.2°, 9.27° ± 0.2°, 9.93° ± 0.2°, 11.06° ± 0.2°, 11.27° ± 0.2°, 12.02° ± 0.2°, 12.82° ± 0.2°, 13.30° ± 0.2°, 14.87° ± 0.2°, 16.62° ± 0.2°, 17.09° ± 0.2°, 18.11° ± 0.2°, 19.32° ± 0.2°, 19.79° ± 0.2°, 20.69° ± 0.2°, 21.22° ± 0.2°, 22.27° ± 0.2°, 23.41° ± 0.2°, 24.38° ± 0.2°, 25.47° ± 0.2°, 26.58° ± 0.2°, 27.43° ± 0.2°, 27.87° ± 0.2°, 28.32° ± 0.2°, 29.45° ± 0.2°, 30.34° ± 0.2°, 31.62° ± 0.2° and 33.79° ± 0.2°; or
wherein the p-toluenesulfonate salt is the crystal form A of the p-toluenesulfonate salt, which is **characterized by** an X-ray powder diffraction pattern comprising peaks expressed as 2θ at 6.49° ± 0.2°, 9.51° ± 0.2°, 10.04° ± 0.2°, 10.51° ± 0.2°, 10.72° ± 0.2°, 11.71° ± 0.2°, 12.65° ± 0.2°, 13.20° ± 0.2°, 14.10° ± 0.2°, 14.35° ± 0.2°, 14.94° ± 0.2°, 15.88° ± 0.2°, 16.44° ± 0.2°, 16.82° ± 0.2°, 17.21° ± 0.2°, 17.86° ± 0.2°, 18.34° ± 0.2°, 19.08° ± 0.2°, 19.57° ± 0.2°, 20.17° ± 0.2°, 20.65° ± 0.2°, 20.84° ± 0.2°, 21.26° ± 0.2°, 21.87° ± 0.2°, 22.27° ± 0.2°, 22.44° ± 0.2°, 22.84° ± 0.2°, 23.19° ± 0.2°, 23.55° ± 0.2°, 24.25° ± 0.2°, 24.81° ± 0.2°, 25.59° ± 0.2°, 26.21° ± 0.2°, 26.91° ± 0.2°, 27.38° ± 0.2°, 27.93° ± 0.2°, 28.26° ± 0.2°, 28.82° ± 0.2°, 29.16° ± 0.2°, 29.61° ± 0.2°, 30.19° ± 0.2°, 31.30° ± 0.2°, 31.90° ± 0.2°, 32.44° ± 0.2°, 33.21° ± 0.2°, 33.94° ± 0.2°, 35.32° ± 0.2°, 36.44° ± 0.2°, 37.30° ± 0.2°, 37.61° ± 0.2°, 38.59° ± 0.2°, 39.54° ± 0.2°, 42.25° ± 0.2°, 44.61° ± 0.2° and 47.32° ± 0.2°.

5. The salt according to any one of claims 1 to 4, wherein the sulfate salt is the crystal form A of the sulfate salt, which is **characterized by** an X-ray powder diffraction pattern substantially as shown in Figure 1;
wherein the bisulfate salt is the crystal form A of the bisulfate salt, which is **characterized by** an X-ray powder diffraction pattern substantially as shown in Figure 4;
wherein the phosphate salt is the crystal form A of the phosphate salt, which is **characterized by** an X-ray powder diffraction pattern substantially as shown in Figure 7; or
wherein the p-toluenesulfonate salt is the crystal form A of the p-toluenesulfonate salt, which is **characterized by** an X-ray powder diffraction pattern substantially as shown in Figure 10.

6. The salt according to any one of claims 1 to 5, wherein the sulfate salt is the crystal form A of the sulfate salt, which is **characterized by** a differential scanning calorimetry thermogram comprising endothermic peaks at 105.07°C ± 3°C and 185.08°C ± 3°C and exothermic peak at 233.00°C ± 3°C;
wherein the bisulfate salt is the crystal form A of the bisulfate salt, which is **characterized by** a differential scanning calorimetry thermogram comprising an endothermic peak at 196.02°C ± 3°C and exothermic peaks at 201.85°C ± 3°C and 246.30°C ± 3°C;
wherein the phosphate salt is the crystal form A of the phosphate salt, which is **characterized by** a differential scanning calorimetry thermogram comprising an exothermic peak at 205.70°C ± 3°C; or
wherein the p-toluenesulfonate salt is the crystal form A of the p-toluenesulfonate salt, which is **characterized by** a differential scanning calorimetry thermogram comprising an endothermic peak at 73.89°C ± 3°C and exothermic peak at 231.68°C ± 3°C.

7. The salt according to any one of claims 1 to 6, wherein the sulfate salt is the crystal form A of the sulfate salt, which is **characterized by** a differential scanning calorimetry substantially as described in Figure 2;
wherein the bisulfate salt is the crystal form A of the bisulfate salt, which is **characterized by** a differential scanning calorimetry substantially as described in Figure 5;
wherein the phosphate salt is the crystal form A of the phosphate salt, which is **characterized by** a differential scanning calorimetry substantially as described in Figure 8; or
wherein the p-toluenesulfonate salt is the crystal form A of the p-toluenesulfonate salt, which is **characterized by** a differential scanning calorimetry substantially as described in Figure 11.

8. The salt according to any one of claims 1 to 7, wherein the sulfate salt is the crystal form A of the sulfate salt, which is **characterized by** an thermogravimetric analysis (TGA) substantially as shown in Figure 3.
wherein the bisulfate salt is the crystal form A of the bisulfate salt, which is **characterized by** an thermogravimetric analysis (TGA) substantially as shown in Figure 6;
wherein the phosphate salt is the crystal form A of the phosphate salt, which is **characterized by** an thermogravimetric analysis (TGA) substantially as shown in Figure 9; or
wherein the p-toluenesulfonate salt is the crystal form A of the p-toluenesulfonate salt, which is **characterized by** an thermogravimetric analysis (TGA) substantially as shown in Figure 12.

9. A pharmaceutical composition comprising the salt according to any one of claims 1 to 8, and a combination thereof of pharmaceutically acceptable carriers, excipients, diluents, adjuvants or composition thereof.

10. Use of the salt according to any one of claims 1-8 or the pharmaceutical composition of claim 9 in the manufacture of a medicament for protection, management, treatment or lessening diseases associated with P13K kinase abnormalities.

11. The use of claim 10, wherein the diseases associated with P13K kinase abnormalities comprises respiratory diseases, viral infections, non-viral respiratory infections, allergic diseases, autoimmune diseases, inflammatory diseases, cardiovascular diseases, hematologic malignancies, neurodegenerative diseases, pancreatitis, multi-organ failure, kidney disease, platelet aggregation, cancer, transplant rejection, lung injury or pain.

12. The use of claim 10, wherein the diseases associated with P13K kinase abnormalities comprises asthma, chronic obstructive pulmonary disease, viral respiratory infections, worsening of viral respiratory diseases, aspergillosis, leishmaniasis, allergic rhinitis, allergic dermatitis, rheumatoid arthritis, multiple sclerosis, inflammatory bowel disease, thrombosis, atherosclerosis, hematologic malignancies, neurodegenerative diseases, pancreatitis, multi-organ failure, nephropathy, platelet aggregation, cancer, transplant rejection, lung injury, systemic inflammatory pain, diabetic neuropathy, chronic lymphocytic leukemia, non-hodgkin's lymphoma, hairy cell leukemia, mantle cell lymphoma, burkitt lymphoma, small lymphocytic lymphoma, follicular lymphoma, lymphoplasmaplasma cell lymphoma, extranodal marginal zone lymphoma, Hodgkin's lymphoma, Waldenström macroglobulinemia, prolymphocytic leukemia, acute lymphoblastic leukemia, myelofibrosis, mucosa-associated lymphoid tissue lymphoma, B-cell lymphoma, thymic mediastinal large B-cell lymphoma, lymphomatoid granulomatous disease, splenic marginal zone lymphoma, primary exudative lymphoma, intravascular large B-cell lymphoma, plasma cell leukemia, extramedullary plasmacytoma, stagonic myeloma, monoclonal gammaglobulinosis or B-cell lymphoma.
